# EUROPÄISCHE PATENTSCHRIFT

(11) **EP 2 210 619 B1**
(45) Veröffentlichungstag und Bekanntmachung des Hinweises auf die Patenterteilung: **20.12.2017**
(21) Anmeldenummer: 10000102.3
(22) Anmeldetag: 08.01.2010
(51) Int. Cl.: A61L 15/42, A61L 15/26

(54) **NARBENABDECKUNG MIT UV-SCHUTZ**
Scar covering with UV protection
Recouvrement de cicatrice avec protection UV

(30) Priorität: 15.01.2009 DE 102009005143
(43) Veröffentlichungstag der Anmeldung: 28.07.2010
(73) Patentinhaber: Beiersdorf AG, 20245 Hamburg (DE)
(72) Erfinder: Hartkopf, Carsten, 22337 Hamburg (DE); Kirsten, Juana, 22301 Hamburg (DE); Meyer, Christiane, 20357 Hamburg (DE); Blohm, Alexandra, 20257 Hamburg (DE)
(74) Vertreter: Hartmann, Jost

(56) Entgegenhaltungen:
- WO-A1-2005/004936
- WO-A2-2005/027859
- DE-A1- 10 212 866
- US-A1- 2003 175 333
- US-A1- 2005 137 272

## Beschreibung

Die Erfindung betrifft Narbenabedeckungen mit integriertem UV-Schutz.

Die Narbenabdeckung umfasst eine Narbenauflage aus einer atmungsaktiven und bevorzugt klebenden Polyurethan-Matrix und gegebenenfalls einer Trägerfolie oder -lack aus einer bevorzugt wasserdampfdurch- und wasserundurchlässiger Polymerschicht. Um Narben vor UV-Strahlung zu schützen, sind in der Narbenauflage und/oder in der Trägerfolie bzw- Lack ein oder mehrere organische UV-Filtersubstanzen enthalten.

In der EP 782457 A1 werden auf Silikongel basierende Narbenabdeckungen beschrieben. In der DE 10212866 A1 werden Narbenreduktionspflaster auf Polyurethanbasis beschrieben. Darin wird ausführlich die Bildung und Pflege von Wunden und den sich dabei bildenden Narben beschrieben.

Hypertrophe Narben sind gegenüber der umliegenden Haut erhaben und zeigen eine Vielzahl an Variationen in Größe, Form, Farbe und Konsistenz. Diese Kennzeichen hängen zum einen von dem Ort und der Größe der Verletzung und zum anderen von der zeitlichen Entwicklung und der persönlichen Anfälligkeit ab.

Im Unterschied zu bekannten Wundversorgungsverbänden und Pflastern werden Narbenverbände über einen längeren Zeitraum auf der Haut belassen um einen gewünschten Reduktionseffekt zu gewährleisten. Die bekannten Narbenabdeckungen sind zum Teil transparent oder transluzent gestaltet um ästhetischen Anforderungen zu genügen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut. Man hat lange Zeit fälschlicherweise angenommen, dass die langweilige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Wie bei allen Verletzungen und Schädigungen der Haut gilt auch für Narbengewebe, dass es besonders empfindlich auf UV-Strahlung reagieren kann. Insbesondere frische hypertrophe Narben und Keloide sind durch die bekannten Narbenauflagen nicht oder nur gering geschützt. Insbesondere sind die Narben nicht vor der UV-A Strahlung hinreichend geschützt. Es kann daher leichter zu Schädigungen des Narbengewebes durch die Sonneneinstrahlung kommen.
In der Folge kann die Haut und Narbe dann mit Farbveränderungen wie einer intensivierten Rötung oder bräunlichen Verfärbung, einer so genannten Hyperpigmentierung, reagieren.

Wünschenswert ist es daher Narben generell - vor allem aber, wenn es sich um frische Narben handelt - vor UV-Strahlung zu schützen.
Der bisherige Rat zielte daher darauf, dass sich der Anwender keiner intensiven Sonneneinstrahlung und/oder Solarium aussetzen sollte oder durch einen entsprechenden UV-Schutz oder schützende Kleidung abdecken sollte. Dies auch, weil applizierte Narbenpflaster über einen längeren Zeitraum getragen werden und die darunter liegende Haut und Narbe nicht mit Sonnencreme geschützt wird.

Da bekannte Narbenpflaster nicht sichtbar sein sollen und somit häufig transparent gestaltet sind, bieten diese keinen wirksamen UV-Schutz. Derzeitige Narbenpflaster weisen nur aufgrund ihrer Abdeckwirkung einen Lichtschutzfaktor von maximal SPF 7 auf. Dies ist jedoch kein ausreichender Schutzfaktor. Ein Lichtschutzfaktor von SPF größer 25 ist medizinisch indiziert.

Die WO 2005027859 beschreibt Pflaster bestehend aus zwei Lagen, wobei eine Lage klebend und eine der beiden Lagen gegenüber UV-Strahlen lichtdicht ausgestaltet ist.

US 5811108 beschreibt auf der Haut selbstklebende Tattoos, die gegenüber UV Licht undurchlässig gestaltet sind.
Polyurethanmatrices umfassend UV-Filter sind in den Dokumenten nicht erwähnt.

In der WO 2005/004936 A1 werden Haut- oder Wundauflagen beschrieben, die wundheilungsfördernde oder hautpflegende Substanzen enthalten. Die hautpflegenden oder wundheilungsfördernden Substanzen sind dabei nicht in einer Polyurethanmatrix enthalten sondern gänzlich in einem wasserabsorbierenden Polymer inkorporiert. Des Weiteren müssen die Substanzen an die Haut abgegeben werden um ihren Hautpflege oder Wundheilung entfalten zu können.

US 2005/0137272 A1 beschreibt gellierte schaumförmige B iopolymere. Diese Polymere können als Wundauflagen, in der Mundhygiene, Fußpflege, Kosmetik oder für die Wirkstoffabgabe genutzt werden.

US 2003/0175333 A1 beschreibt Hautauflage für die kontrollierte topische Wirkstofffreigabe.

Des Weiteren wird durch bekannte Pflasterabdeckungen oder Kleidung nur die UV B-Strahlung absorbiert. Jedoch hat UV A-Strahlung einen wesentlich schädlicheren Einfluss auf frisches Narbengewebe (Hyperpigmentierung), so dass es auch die Aufgabe der vorliegenden Erfindung ist, den UV-Schutz von Narben im Bereich UV-B als auch UV-A sicher zu stellen.

Eine Aufgabe der vorliegenden Erfindung ist es daher die Nachteile des Standes der Technik zu beseitigen. Insbesondere ist es die Aufgabe der vorliegenden Erfindung eine Narbenabdeckung bereit zu stellen, die eine Rückbildung hyperthroper Narben ermöglicht, eine hautschonende, langanhaltende Klebung gewährleistet, schmerzfrei und rückstandslos ablösbar ist und insbesondere einen UV Schutz für die abgedeckte Narbe bzw. Haut zu gewährleisten.

Bekannt ist eine Narbensalbe (Mederma, Fa. Merz) mit SPF 30 die im Handel erhältlich ist. Bekannt sind des Weiteren Gegenstände aus Kunststoff oder anderen Polymermaterialien, die aus Gründen der Farbstabilisierung UV-Schutzsubstanzen enthalten. Nachteilig ist, dass bei Gegenständen aus Kunststoff, die der Sonnenbestrahlung ausgesetzt sind, diese mit der Zeit vergilben. Als Lösung dieses Problems setzt man den Kunststoffen Lichtschutzsubstanzen zu.

Die in Alltagsgegenständen enthaltenen UV Schutzsubstanzen sind jedoch zum Teil nicht für Kosmetik- oder Medizinprodukte zugelassen, so dass diese nicht für die erfindungsgemäße Anwendung geeignet sind.

Aus all diesen Informationen des Standes der Technik ist der Weg zur vorliegenden Erfindung nicht vorgezeigt gewesen.

Gelöst werden die Aufgaben mit einer Narbenabdeckung entsprechend den Hauptansprüchen. Gegenstand der Unteransprüche sind vorteilhafte Ausführungsformen der erfindungsgemäßen Abdeckung.

Die Erfindung umfasst Narbenabdeckungen, umfassend eine Narbenauflage aus einer atmungsaktiven Matrix aus Polyurethan, und gegebenenfalls einer Trägerfolie aus Polyurethan. In der Narbenauflage und/oder in der Trägerfolie sind ein oder mehrere organische UV-Filtersubstanzen enthalten.

Die Narbenauflage besteht vorteilhaft aus Polyurethan und kann eine Dicke von 0,2 bis 2 mm, insbesondere von 0,5 bis 1,5 mm, aufweisen.

Die Polyurethanauflage ist bevorzugt klebend ausgestaltet. Bevorzugte Ausführungsformen, Gestalt und Größe der Polyurethanauflage sind in der DE 10212866 dargestellt. Geeignete Polyurethan-Xerogele als Matrix sind Gegenstand der DE 19618825, in der hydrophile, selbstklebende Polyurethan-Gele offenbart werden. Weiter vorzugsweise kommen Polyurethan-Xerogele zum Einsatz, wie sie in der EP 0 665 856 B1 offenbart sind. Die Polyurethanmatrix kann auch aus den an sich aus der Polyurethanchemie bekannten Ausgangsverbindungen nach an sich bekannten Verfahren hergestellt werden, wie sie zum Beispiel in DE 31 03 499 A1, DE 31 03 500 A1 und EP 0 147 588 A1 beschrieben sind.

Zur Modifizierung der Hafteigenschaften der Narbenauflage können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Matrixmasse, zugegeben werden. Damit ist der Begriff - Narbenauflage bestehend aus Polyurethan - weiterhin zutreffend.

Die Polyurethanschicht der Narbenauflagenmatrix ist insbesondere transparent, wasserdampfdurchlässig und klebend. Dies stellt einen signifikanten vorteilhaften Unterschied zu den Silikongel basierenden Narbenauflagen dar. Die Transparenz erhöht zudem die Akzeptanz beim Anwender, da ein Narbenreduktionspflaster üblicherweise über einen längeren Zeitraum auf der Haut getragen wird.

In der transparenten Ausführungsform wird zudem der UV-Schutz als zwingend notwendig vom Anwender anerkannt.

Zur Speicherung von Hautflüssigkeit kann vorzugsweise ein Superabsorber-Polymer als Pulver in der Matrix eingearbeitet werden.

Es ist daher von Vorteil in die Narbenauflage Superabsorber oder superabsorbierendes Polymer in einer Menge von 0,01 bis 30 Gew.%, insbesondere von 0,5 bis 25 Gew.%, insbesondere 10 Gew.%, bezogen auf die Gesamtmasse der Matrix, einzuarbeiten.

Polyurethane erweisen sich gegenüber anderen Klebematerialen, wie Polyacrylaten, Kautschuk etc., als äußerst vorteilhaft, da sie kein bekanntes Allergiepotential beinhalten.
Des Weiteren sind die bei der Einarbeitung von UV-Filtern in Polyurethanmatrices zu erwartenden Nachteile bei der Matrixbildung ausgeblieben, wie die nachfolgenden Herstellbeispiele zeigen.

Eine Selbstklebung der Abdeckung ist bevorzugt, da somit eine einfache Ausführungsform gestaltet werden kann und der Zweck der Narbenreduktion auf effiziente Weise genüge getan wird. D.h. eine Schicht aus selbstklebender PU-matrix umfassend UV-Filter wird den erfindunsgemäßen Aufgaben gerecht.

Ist die Auflage nicht selbstklebend formuliert, kann die Abdeckung als Tuch oder Pad als ebensolch wirksame Narbenlichtschutzabdeckung verwendet werden.
Der Anwender kann selbstverständlich die nicht selbstklebende Variante der erfindungsgemäßen Abdeckung mit zusätzlichen Heftklebestreifen auf der Haut fixieren.

Vorteilhaft beträgt die Dicke der Trägerfolie ca. 10 bis 100 µm. Die u.U. flexible Trägerfolie besteht aus Polymeren von Polyurethan.
Die Trägerfolie kann über gießen, extrudieren und blasen erfolgen. Lieferanten bekannter Trägerfolien sind etablierte Folienhersteller, wie beispielsweise die Firmen SNEF oder Epurex. Die Trägerfolie kann erfindungsgemäß auch als Lackschicht formuliert werden.
Diese Lackschicht wird auf die PU.Schicht aufgetragen.
Als geeignete Lacke kommen die aus der Kosmetik und Dermatologie bekannten Lacke bevorzugt zum Einsatz, insbesondere transparente Lacke.
Bekannt sind beispielsweise Polyurethanlacke

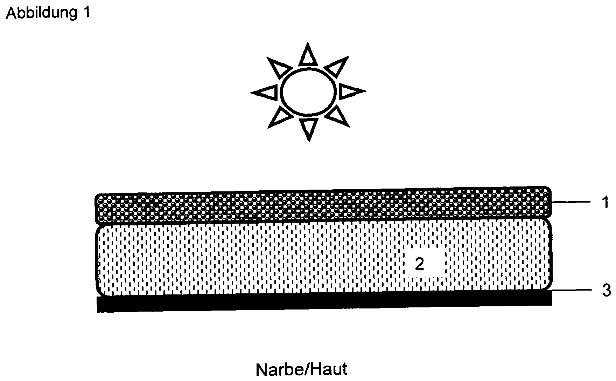

Als vorteilhafte Ausführungsform umfasst die Narbenabdeckung eine selbstklebende Polyurethannarbenauflage ohne UV Filter und einer darauf angebrachten Polyurethanträgerfolie, in die der oder die UV Filter eingebracht sind. Abbildung 1 veranschaulicht diese vorteilhafte Narbenabdeckung schematisch. Die Trägerfolie aus Polyurethan (1) umfasst ein oder mehrere UV-Filter. Die Trägerfolie ist auf einer selbstklebenden Polyurethanmatrix (2) aufgebracht. Zum Transport und Lagerung ist die klebende Seite der Abdeckung mit einem Release Liner (3) abgedeckt, der vor dem Applizieren auf die Haut/Narbe entfernt wird. Es wird so über einen längeren Zeitraum sowohl Narbenreduktion mit gleichzeitigem UV Schutz gewährleistet.

Als UV-Filtersubstanzen können ein oder mehrere der bekannten UV-Filter eingesetzt werden, wie sie beispielsweise aus der DE 10155963 bekannt sind.
Erfindungsgemäß vorteilhafte Ausführungsformen sind dadurch gekennzeichnet, dass die Abdeckung einen oder mehrere UV-Filter gewählt aus der Gruppe der Verbindungen 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; Ethylhexylsalicylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenyl-acrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin, Merocyanine umfasst.

Bevorzugt werden ein oder mehrere Filter eingesetzt, die in Anlage 7 der Kosmetik VO aufgeführte organische Lichtschutzfilter aufgeführt sind, wie sie in der nachfolgenden Tabelle aufgeführt sind.

| **Chemische Bezeichnung** | **INCI-Bezeichnung** | **Handelsnamen® (Beispiele)** | **Wirkspektrum** |
|---|---|---|---|
| 4-Aminobenzoesäure | PABA | PABA | UVB |
| 3-(4'Trimethylammonium)-Benzylidenbornan-2-on-methylsulfat | Camphor Benzalkonium Methosulfate | Mexoryl SK | UVB |
| 3,3,5-Trimethyl-cyclohexylsalicylat | Homosalate | Eusolex, HMS Neo Heliopan | UVB |
| 2-Hydroxy-4methoxybenzophenon | Benzophenone-3 | Eusolex 4360 Neo Heliopan BB Uvinul M40 Escalol 567 UVAsorb MET/C | UVA/UVB |
| 2-Phenylbenzimidazol-5-sulfonsäure und ihre Kalium-, Natrium- und Triethanolaminsalze | Phenylbenzimidazole Sulfonic Acid (PBSA) | Eusolex 232 Neo Heliopan Hydro Parsol HS | UVB |
| 3,3'-(1,4-Phenylendiaethin)-bis-(7,7-dimethyl-2-oxobicyclo-[2.2.1]-heptan-1-methansulfonsäure)und ihre Salze | Terephthalidene Dicamphor Sulfonic acid (TDSA) | Mexoryl SX | UVA |
| 1-(4-tert.Butylphenyl)-3-(4methoxyphenyl)propan-1,3-dion | Butyl Methoxydibenzoylmethane (BMDM) | Eusolex 9020 Parsol 1789 | UVA |
| 3-(4'-Sulfo)-benzylidenbornan-2-on und seine Salze | Benzylidene Camphor Sulfonic Acid | Mexoryl SL | UVB |
| 2-Cyan-3,3-diphenylacrylsäure (2-ethylhexylester) | Octocrylene (OC) | Eusolex OCR Neo Heliopan 303 Uvinul N-539 | UVB |
| Polymer von N-[2(und4)-(2-oxobom-3-ylidenmethyl)benzyl]-acrylamid | Polyacryl-amidomethyl Benzylidene Camphor | Mexoryl SW | UVB |
| 4-Methoxy-zimtsäure-2-ethylhexylester | Octyl Methoxycinnamate (EHMC) | Escalol 557 Eusolex 2292 Neo Heliopan AV Parsol MCX | UVB |
| Ethoxyliertes Ethyl-4-aminobenzoat | PEG-25-PABA | Uvinul P 25 Unipabol U 17 | UVB |
| 4-Methoxy-zimtsäureisoamylester | Isoamyl-Methocycinnamate (IMC) | Neo Heliopan E 1000 | UVB |
| 2,4,6-Tris[p-(ethylhexyloxycarbonyl) anilino]-1,3,5-triazin | Octyl Triazone | Uvinul T 150 | UVB |
| 2-(2H-benzotriazol-2-yl)-4-methyl-6-(2-methyl-3-(1,3,3,3-tetramethyl-1-(trimethylsilyloxy)-disiloxanyl)propyl) phenol | Drometrizole Trisiloxane (DTS) | Mexoryl XL | UVA/UVB |
| 4,4'-[(6-[4-((1,1-Dimethylethyl)-aminocarbonyl)phenylamino]-1,3,5-triazin-2,4-yl)diimino]bis-(benzoesäure-2-ethylhexylester) | Dioctyl Butamido Triazone (DEBT) | UVAsorb HEB | UVA |
| 3-(4'-Methylbenzyliden)-DL-campher | 4-Methylbenzylidene Camphor (MBC) | Eusolex 6300 Neo Heliopan MBC Parsol 500 | UVB |
| 3-Benzyliden-campher | 3-Benzylidene Camphor | Mexoryl SD-20 Unisol-S-22 | |
| Salicylsäure-2-ethylhexylester | Octylsalicylat (EHS) | Escalol 587 Neo Heliopan OS | UVB |
| 4-Dimethylaminobenzoesäure-2-ethylhexylester | Octyl dimethyl PABA | Escalol 507 Eusolex 6007 | UVB |
| 2-Hydroxy-4-methoxybenzophenon-5-sulfonsäure und das Natriumsalz | Benzophenone-4 | Uvasorb S 5 Escalol 577 Uvinul MS 40 | UVA/UVB |
| 2,2'-Methylen-bis(6-(2H-benzotriazol-2-yl)-4-(1,1,3,3-tetramethylbutyl)phenol) | Methylen bis-Benzotriazolyl Tetramethylbutylphenol (MBBT) | Tinosorb M | UVA/UVB |
| 2,2'-(1,4-Phenylen)bis(1 H-benzimidazol-4,6-disulfonsäure, Mononatriumsalz) | Disodium Phenyl Dibenzimidazole Tetrasulfonate (DPDT) | Neo Heliopan AP | UVA/UVB |
| 2,4-Bis[4-(2-ethylhexyloxy)-2-hydroxyphenyl]-6-(4-methoxyphenyl)-1,3,5-triazen | Bis-Ethylhexyloxyphenol Methoxyphenyltriazin (BEMT) | Tinosorb S | UVA/UVB |

Die UV-Filter, ggf. als Feststoff gelöst in einem Lösemittel, werden bevorzugt in der entsprechenden erlaubten Konzentration gemäß Cosmetic Directive, Annex VII/I,25(S81) dem jeweiligen Polymergranulat zugemischt, erhitzt und die entstehende Masse wird als Folie ausgestrichen bzw. im Extruder dem Polymergranulat zugesetzt und im weiteren zur Folie verarbeitet.

Die als Narbenauflage formulierte Matrix kann ebenso als Folie hergestellt werden.

Es ist vorteilhaft im Sinne der vorliegenden Erfindung, wenn die öllöslichen UV-Filter durch Lipide in Lösung gebracht werden. Die Lipide werden vorteilhaft gewählt aus der Gruppe der Verbindungen Butylenglycol Dicaprylat/Dicaprate, Octyldodecanol, C12-15 Alkyl Benzoat, Caprylic/Capric Triglyceride, Diisopropyl Sebacate, Dicaprylyl Ether, Mineralöl und/oder Silikonöl.
Ferner können die festen UV-Filter durch Lösemittel wie Ethanol gelöst werden.
Die wasserlöslichen UV-Filter wie Phenylbenzimidazole Sulfonic Acid können als Salze in Lösung gebracht werden
Es ist vorteilhaft im Sinne der vorliegenden Erfindung, wenn die öllöslichen UV-Filter durch folgende Lipide in Lösung gebracht werden. Die Lipide werden vorteilhaft gewählt aus der Gruppe der Verbindungen Butylenglycol Dicaprylat/Dicaprate, Octyldodecanol, C12-15 Alkyl Benzoat, Caprylic/Capric Triglyceride, Diisopropyl Sebacate, Dicaprylyl Ether, Mineralöl und/oder Silikonöl.
Ferner können die festen UV-Filter durch Lösemittel wie Ethanol gelöst werden.
Die wasserlöslichen UV-Filter wie Phenylbenzimidazole Sulfonic Acid können als Salze in Lösung gebracht werden.
Die in Lösung befindlichen UV Filter werden in gleicher Weise der Matrix bzw. Folie zugegeben.

Bevorzugt einzusetzende UV-Filter sind Butyl Methoxydibenzoylmethane (Parsol 1789), Disodium Phenyl Dibenzimidazole Tetrasulfonate (Neo Heliopan AP), Octocrylene (Neo Heliopan 303), Ethylhexyl Triazone (Uvinul T 150), Ethylhexyl Salicylate (Neo Heliopan OS), Phenylbenzimidazole Sulfonic Acid (Eusolex 232),Ethylhexyl Methoxycinnamate + BHT (Uvinul MC 80, Homosalate (Eusolex HMS), Benzophenone-3 und/oder Bis-Ethylhexyloxyphenol Methoxyphenyl Triazine (Tinosorb S), 4-(Dimethylamino)-benzoesäure(2-ethylhexyl)ester (Uvinul A Plus).

Da sowohl der UV A- als auch der UV B-Bereich vorteilhaft abzudecken sind, wird entweder eine Mischung aus zwei UV-Filtern oder ein Breitbandfilter erfindungsgemäß bevorzugt eingesetzt.
Vorteilhaft werden aus folgenden Filtern mindestens ein UV-A und mindestens ein UV-B Filter als Mischung eingesetzt:
UV A: Butyl Methoxydibenzoylmethane (0,1 - 5%), Disodium Phenyl Dibenzimidazole Tetrasulfonate (0,1 - 10%), Octocrylene (0,1 - 10%)
UV B: Ethylhexyl Triazone (0,1 - 5%), Ehtylhexyl Salicylate,Phenylbenzimidazole Sulfonic Acid (0,1 - 8%), Ethylhexyl Methoxycinnamate + BHT (0,1 - 10%), Homosalate (0,1 - 10%). Bevorzugt werden auch sogenannte Breitbandfilter (UV A + B) wie Benzophenone-3 (0,1 - 10%), Benzophenone-3 und/oder Bis-Ethylhexyloxyphenol Methoxyphenyl Triazine (0,1 - 10%). In Klammern sind die bevorzugten Gewichtsanteile der Filtersubstanzen, bezogen auf die Gesamtmasse der sie enthaltenden Matrix bzw. Folie, angegeben.

Die Eigenfärbung mancher UV-Filter ist eine spezifische Stoffeigenschaft, die nicht angepasst werden kann. Durch Mikronisierung kann die Sichtbarkeit der Färbung in der Matrix bzw. der Folie unterdrückt werden. Unter Mikronisieren wird die Reduktion der Partikelgröße auf weniger als 10 µm, insbesondere durch Mahlprozesse, verstanden. Dadurch werden die UV-Filter einerseits leistungsfähiger und interessanter für Ihre speziellen Anwendungen und gleichzeitig wird die Eigenfärbung unterdrückt.

Die UV-Schutzleistung der Abdeckung bzw. der ihnen zugrunde liegenden UV-Filter wird in der Regel in biologischen Wirksamkeitsprüfungen unter standardisierten Bedingungen bestimmt. Mit "UV-Schutzleistung" ist im Sinne der vorliegenden Erfindung sowohl die Schutzleistung gegenüber UV-A-Strahlung als auch gegenüber UV-B-Strahlung gemeint.

Ein Maß für die UV-Schutzleistung stellen im Sinne der vorliegenden Erfindung beispielsweise der Lichtschutzfaktor (LSF bzw. SPF) oder auch IPD-Werte und dergleichen dar.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muss die Zubereitung mindestens 90 % der UV-A-Strahlung im Bereich von 320 bis 360 nm absorbieren.

Die erfindungsgemäße Narbenabdeckung schützt frisches Narbengewebe verlässlich und allumfassend vor schädlichen äußeren Einflüssen, ohne dass sich der Anwender Gedanken mache müsste, auf welche Gefährdungen er bzgl. seiner frischen Narbe zu achten hätte. Gleichzeitig wird die Wirkung als Narbenpflasters hinsichtlich Reduktion der Rötung verbessert werden, da erfindungsgemäß keine die Hyperpigmentierung fördernde UV-Strahlung mehr das Narbengewebe schädigen kann.

Des Weiteren bietet die erfindungsgemäße Narbenabdeckung, bevorzugt als selbstklebendes Narbenpflaster formuliert, mit hohem SPF auf bequeme und wirksame Weise eine narbenreduzierende Wirkung und gleichzeitig einen UV-Schutz in einem einzigen Produkt.
Durch die erfindungsgemäße Kombination aus Polyurethanmatrix, die nachweisliche eine Narbenreduktionswirkung hat, und UV-Filtern werden zwei für die Narbenbehandlung wesentlichen vorteilhaften Wirkungen verbunden.

Es existieren zwar Narbenpflaster mit nachgewiesener Narbenreduktionswirkung, aber ohne medizinisch akzeptablem UV Schutz (SPF > 25). Es existieren des Weiteren Sonnenschutzcremes, die aber keine narbenreduzierende Wirkung haben. Eine gleichzeitige Anwendung beider Produktarten ist bislang nicht möglich, da ein Narbenpflaster auf der eingecremten Haut nicht haftet.
An der bekannten Narbencreme mit SPF 30 ist nachteilig, dass die Anwendung einer Creme von vielen Konsumenten nicht bevorzugt ist, da die Hände danach fettig sind. Des Weiteren ist eine Narbencreme mehrmals am Tag anzuwenden, wohingegen ein Narbenpflaster einen Tag bis mehrere Tage getragen werden kann. Außerdem bietet die erfindungsgemäße Narbenabdeckung mit hohem SPF einen über die gesamte Tragedauer anhaltenden UV-Schutz, wohingegen bei einer Creme nach einiger Zeit nachgecremt werden muss.

Vorteilhaft ist die erfindungsgemäß bevorzugte Narbenabdeckung so formuliert, dass der oder die UV-Filter in die Trägerfolie eingearbeitet sind. Die Trägerfolie kann in einer bevorzugten Ausführungsform als Lack formuliert sein.
In einer weiteren bevorzugten Ausführungsform besteht die Trägerfolie vorteilhaft aus Polyurethan (PU-Folie).

Die erfindungsgemäße Narbenabdeckung ermöglicht so eine Rückbildung hyperthropher Narben. Als selbstklebendes PU-Pflaster ist eine hautschonende, langanhaltende Klebung gewährleistet, die schmerzfrei und rückstandslos ablösbar ist. Durch die integrierten UV Filter ist ein UV Schutz für die abgedeckte Narbe bzw. Haut gewährleistet. Der SPF der erfindungsgemäßen Abdeckung kann über 25 liegen, wenn beispielsweise die bevorzugt genannten UV Filter jeweils zu einem Anteil von bis zu 10 Gew.%, bezogen auf die Gesamtmasse der Matrix bzw. Folie, eingesetzt werden. Bevorzugt werden Mischungen aus zwei oder drei UV-Filtern eingesetzt.

In einer bevorzugten Ausführungsform ist die Randschicht der Narbenauflage zum Rand auf eine Dicke von maximal 5 bis 150 µm, insbesondere 30 bis 90 µm, abgeschrägt, wie es in der DE 10212866 beschrieben ist.
Dies hat den Vorteil, dass als Pflaster formuliert, die Aufrollneigung, wie sie übliche Pflaster besitzen, verursacht durch Kleidung, Bettwäsche, Hautkontakt oder beim Waschen, stark reduziert ist. Das wiederum ermöglicht erst die für die Narbenbehandlung lange Tragedauer des Narbenpflasters ohne zusätzliche Fixierungen.

Die erfindungsgemäße Narbenabdeckung kann eine längliche, aufrollbare und beliebig ablängbare Form besitzen. Das hat den Vorteil, dass der Anwender von der Rolle eine entsprechend der individuellen Narbenlänge angepasste, einstückige Abdeckung zurecht schneiden kann.

Die Abdeckung kann zum besseren, sterileren Transport und Lagerung mit einer Schutzabdeckung (release liner) auf der bevorzugt klebenden Seite ausgestattet sein, die vor dem Anlegen der Abdeckung auf die Haut entfernt wird. Diese kann beispielsweise silikonisiertes Papier oder eine insbesondere silikonisierte Folie sein, so dass die klebende Seite während der Lagerung geschützt ist.

Die erfindungsgemäße Abdeckung ist bevorzugt als Narbenreduktionspflaster mit integriertem UV Schutz zu verwenden.

Weiterer Vorteil der erfindungsgemäßen Pflaster ist die Herstellung der Narbenauflage mit/ohne Randschicht aus einem Guss. Es ergeben sich stufenlose Übergänge, die eine variable Form und Größe der Pflaster bei der Herstellung erlauben. Damit ist sowohl die Verankerung der PU-Matrix auf der Trägerfolie als auch die Haftung auf der Haut verbessert.

So ist die Narbenauflage mittig gelegen und geht stufenlos in die Randschicht über.

Das Verfahren zur Herstellung der bevorzugt klebend formulierten Narbenabdeckung aus Polyurethan verläuft vereinfacht über folgende Verfahrensschritte.

Der oder die UV-Filter werden, wie zuvor ausgeführt dem jeweiligen Polymergranulat zugemischt und extrudiert. Da es sich um eine PU-Matrix handelt, werden bevorzugt folgende drei Stufen ablaufen:
a) Aufbringen einer Schicht eines Polyurethangels auf einen Träger (Substrat),
b) Zusammenkaschieren des nicht ausgehärteten Polyurethangels auf dem Träger (Substrat) und einer Trägerfolie insbesondere von einem Rakel und
c) anschließendes Zusammenführen des Laminats in einen Walzenspalt, wo das Polyurethan in die endgültige Pflasterform mit der gewünschten Randschicht ausgewalzt wird.

Der Walzenspalt kann durch eine Glattwalze und eine Konturenwalze gebildet werden. Oder aber die Konturierung erfolgt nach dem Laminieren mittels einer nachgeschalteten Konturenwalze.

Die Konturenwalze weist Aussparungen auf, die eine Herstellung von gewölbten, zum Rand stufenlos dünner werdenden Pflasterformen gewährleisten.

Durch das Herstellverfahren wird die erfindungsgemäße Herstellung von ein- oder mehrschichtigen, naturgemäß dreidimensional konturierten Produkten ermöglicht. Der Narbenauflagenbereich kann dabei einer herkömmlichen Form entsprechen (eine auf das Trägermaterial aufgebrachte erhabene zum Beispiel rechteckige Wundauflage oder beispielsweise linsenförmig gekrümmt sein, mit nach außen konvexer Form, oder aus kombinierten konvexen und konkaven Elementen, wobei die narbenzugewandte Fläche plan ist.

In der Konturenwalze können verschiedene Einprägungen, Aussparungen vorhanden sein, um eine Formung der Polyurethanschicht zu erzielen. Beispielsweise können diese die Form einer halbkonkaven Linse aufweisen. Dies führt in der Polyurethanschicht zu erhöhten Zentren, die sich zum Rand hin abschrägen. Möglich sind auch Ellipsoide, Quader, Würfel oder andere geometrische Formen, um spezielle Formen in der Polyurethanschicht zu erzeugen.

Der Konturierungsverlauf vom Zentrum zum Rand wird durch die gewählte Form festgelegt, das heißt, die Konturenwalze bestimmt das Design der Kontur.

Die Geometrien der herstellbaren konturierten Formkörper sind vielfältig (rund, elliptisch, quadratisch, dreieckig etc.).

Erfindungsgemäß erlaubt das Verfahren einen kontinuierlichen Herstellprozess. Damit sind auch Einsparpotentiale bei der Prozessenergie und den Anlageninvestitionen zu generieren.

Zum Unterschied zu den bekannten Sonnenschutzmitteln in Form von kosmetischen Sprays oder topisch applizierbaren Zubereitung in Form von Emulsionen, Gelen oder Fluiden, sind die erfindungsgemäßen Abdeckungen hochviskos, d.h. sie sind nicht verstreichbar bzw. fließfähig. Die erfindungsgemäßen Narbenabdeckungen sind formbeständige flächige Gebilde, die ohne Änderung der Gestalt appliziert und wieder entfernt werden. Die formbeständigen flächigen Formen sind die Unterscheidungsmerkmale zu den bekannten Sonnenschutzmitteln. Sie wird nicht durch äußere Einflüsse wie Wassereinwirkung, Reibung an anderen Oberflächen (Kleidung, Haut, ...) abgetragen. Die erfindungsgemäße Abdeckung bietet somit einen langanhaltenden verlässlichen Schutz.

Folgende Beispiele zeigen vorteilhaft selbstklebende Polyurethannarbenreduktionspflaster mit hohem UV Schutz.

Dazu wird vorzugsweise die selbstklebende PU-Matrix auf dem Release Liner ausgestrichen, anschließend die PU-Folie, welche folgendermaßen hergestellt wurde - Polyurethan-Granulat wird mit folgenden Beispielvarianten an UV-Filter-Formulierung (siehe Tabelle) homogen vermischt und anschließend zu einer Folie extrudiert - aufgebracht und zu einer Mutterrolle aufgerollt. Diese kann in einem weiteren Verarbeitungsschritt in die gewünschten Pflasterformen (z.B. 7,5 x 4,5 cm) geschnitten werden und in Siegelpapier verpackt.

| Beispiel | 1 | 2 | 3 | 4 | 5 |
|---|---|---|---|---|---|
| Phenylbenzimidazole Sulfonic Acid | x | x | x | | |
| Disodium Phenyl Dibenzimidazole Tetrasulfonate | x | | | | |
| Butyl Methoxydibenzoylmethane | | x | | x | |
| Benzophenone-3 und/oder Bis-Ethylhexyloxyphenol Methoxyphenyl Triazine | | x | x | x | x |
| Octocrylene | | | | x | x |
| 4-(Dimethylamino)-benzoesäure(2-ethylhexyl)ester | | | x | | x |

Die Konzentrationen der eingesetzten UV-Filter liegen jeweils im Bereich 0,1 - max. 10 Gew.%, bezogen auf die Gesamtmasse der Folie.

Als UV-Filter können die in den Beispielen dargestellten gewählt werden.

## Patentansprüche

1. Formbeständige flächige Narbenabdeckung mit integriertem UV-Schutz umfassend eine Narbenauflage aus einer atmungsaktiven Matrix und gegebenenfalls einer Trägerfolie aus einer Polymerschicht **dadurch gekennzeichnet, dass** die Narbenauflage_und Trägerfolie aus Polyurethan bestehen und in der Narbenauflage und/oder in der Trägerfolie ein oder mehrere organische Filtersubstanzen enthalten sind.

2. Abdeckung nach Anspruch 1 umfassend eine Narbenauflage und eine Trägerfolie.

3. Abdeckung nach einem der vorstehenden Ansprüche **dadurch gekennzeichnet, dass** die Narbenauflage selbstklebend ist.

4. Abdeckung nach einem der vorstehenden Ansprüche **dadurch gekennzeichnet, dass** die Trägerfolie wasserdampfdurch- und wasserundurchlässig ist.

5. Abdeckung nach einem der vorstehenden Ansprüche **dadurch gekennzeichnet, dass** die Abdeckung eine längliche, aufrollbare und beliebig ablängbare Form besitzt.

6. Abdeckung nach einem der vorstehenden Ansprüche **dadurch gekennzeichnet, dass** die gesamte Abdeckung transparent ist.

7. Abdeckung nach einem der vorstehenden Ansprüche umfassen ein oder mehrere UV Filter gewählt aus der Gruppe Butyl Methoxydibenzoylmethane, Disodium Phenyl Dibenzimidazole Tetrasulfonate, Octocrylene, Ethylhexyl Triazone, Ethylhexyl Salicylate, Phenylbenzimidazole Sulfonic Acid,Ethylhexyl Methoxycinnamate + BHT, Homosalate, Benzophenone-3 und/oder Bis-Ethylhexyloxyphenol Methoxyphenyl Triazine, 4-(Dimethylamino)-benzoesäure(2-ethylhexyl)ester.

8. Abdeckung nach einem der vorstehenden Ansprüche **dadurch gekennzeichnet, dass** der oder die UV Filter je zu einem Anteil von bis 10 Gew.%, bezogen auf die Gesamtmasse der Matrix oder Folie, enthalten sind.

9. Abdeckung nach einem der vorstehenden Ansprüche **dadurch gekennzeichnet, dass** Mischungen aus zwei oder drei UV Filtern gewählt werden.

10. Abdeckung nach einem der Ansprüche 1 bis 9 zur Herstellung eines Narbenreduktionspflasters mit gleichzeitigem UV Schutz.

## Claims

1. Dimensionally stable, sheet-like scar covering with integrated UV protection, comprising a scar contact material composed of a breathable matrix and optionally a backing film composed of a polymer layer, **characterized in that** the scar contact material and backing film consist of polyurethane, and one or more organic UV filter substances are comprised in the scar contact material and/or in the backing film.

2. Covering according to Claim 1, comprising a scar contact material and a backing film.

3. Covering according to either of the preceding claims, **characterized in that** the scar contact material is self-adhesive.

4. Covering according to any of the preceding claims, **characterized in that** the backing film is water-vapour-permeable and water-impermeable.

5. Covering according to any of the preceding claims, **characterized in that** the covering possesses an elongate shape which can be rolled up and removed in any desired length.

6. Covering according to any of the preceding claims, **characterized in that** the entire covering is transparent.

7. Covering according to any of the preceding claims, comprising one or more UV filters selected from the group consisting of Butyl Methoxydibenzoylmethane, Disodium Phenyl Dibenzimidazole Tetrasulfonate, Octocrylene, Ethylhexyl Triazone, Ethylhexyl Salicylate, Phenylbenzimidazole Sulfonic Acid, Ethylhexyl Methoxycinnamate + BHT, Homosalate, Benzophenone-3 and/or Bis-Ethylhexyloxyphenol Methoxyphenyl Triazine, 2-Ethylhexyl 4-(Dimethylamino)benzoate.

8. Covering according to any of the preceding claims, **characterized in that** the UV filter or filters are each present in a fraction of up to 10% by weight, based on the total mass of the matrix or film.

9. Covering according to any of the preceding claims, **characterized in that** mixtures of two or three UV filters are selected.

10. Covering according to any of Claims 1 to 9 for producing a scar-reducing plaster with simultaneous UV protection.

## Revendications

1. Recouvrement plat, de forme stable, de cicatrice, présentant une protection UV intégrée, comprenant un revêtement de cicatrice en une matrice respirante et le cas échéant une feuille support en une couche polymère, **caractérisé en ce que** le revêtement de cicatrice et la feuille support sont en polyuréthane et une ou plusieurs substances filtre des UV, organiques, sont contenues dans le revêtement de cicatrice et/ou dans la feuille support.

2. Recouvrement selon la revendication 1, comprenant un revêtement de cicatrice et une feuille support.

3. Recouvrement selon l'une quelconque des revendications précédentes, **caractérisé en ce que** le revêtement de cicatrice est auto-adhésif.

4. Recouvrement selon l'une quelconque des revendications précédentes, **caractérisé en ce que** la feuille support est perméable à la vapeur d'eau et imperméable à l'eau.

5. Recouvrement selon l'une quelconque des revendications précédentes, **caractérisé en ce que** le recouvrement présente une forme allongée, pouvant être enroulée et pouvant être découpée de manière quelconque.

6. Recouvrement selon l'une quelconque des revendications précédentes, **caractérisé en ce que** l'ensemble du recouvrement est transparent.

7. Recouvrement selon l'une quelconque des revendications précédentes, comprenant un ou plusieurs filtres UV choisis dans le groupe formé par le butylméthoxydibenzoylméthane, le tétrasulfonate disodique de phényldibenzimidazole, l'octocrylène, l'éthylhexyltriazone, le salicylate d'éthylhexyle, l'acide phénylbenzimidazolesulfonique, le méthoxycinnamate d'éthylhexyle + BHT, l'homosalate, la benzophénone-3 et/ou la bis-éthylhexyloxyphénol-méthoxyphényl-triazine, l'ester 2-éthylhexylique de l'acide 4-(diméthylamino)-benzoïque.

8. Recouvrement selon l'une quelconque des revendications précédentes, **caractérisé en ce que** le ou les filtres UV est/sont contenu(s) à chaque fois en une proportion allant jusqu'à 10% en poids, par rapport à la masse totale de la matrice ou de la feuille.

9. Recouvrement selon l'une quelconque des revendications précédentes, **caractérisé en ce qu'**on choisit des mélanges de deux ou de trois filtres UV.

10. Recouvrement selon l'une quelconque des revendications 1 à 9 pour la préparation d'un pansement de réduction de cicatrice présentant simultanément une protection contre les UV.
